(19) Europäisches Patentamt
European Patent Office
Office européen des brevets

(11) EP 1 616 576 A1

(12) EUROPEAN PATENT APPLICATION
published in accordance with Art. 158(3) EPC

(43) Date of publication:
**18.01.2006 Bulletin 2006/03**

(21) Application number: **04726653.1**

(22) Date of filing: **08.04.2004**

(51) Int Cl.:
***A61K 45/00*** *(1985.01)* ***A61K 31/502*** *(2000.01)*
***A61P 3/10*** *(2000.01)* ***A61P 3/04*** *(2000.01)*
***A61P 3/06*** *(2000.01)* ***A61P 9/00*** *(2000.01)*
***A61P 9/10*** *(2000.01)* ***A61P 9/04*** *(2000.01)*
***A61P 43/00*** *(2000.01)* *C07D 403/04 (1990.01)*
*C07D 237/26 (1985.01)*

(86) International application number:
**PCT/JP2004/005065**

(87) International publication number:
**WO 2004/089412 (21.10.2004 Gazette 2004/43)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **08.04.2003 JP 2003103576**

(71) Applicant: **Mitsubishi Pharma Corporation**
**Osaka-shi,**
**Osaka 541-0046 (JP)**

(72) Inventors:
- **YAMAMOTO, Toshihiro,**
  **c/o MITSUBISHI PHARMA CORP.**
  **Chuo-ku,**
  **Tokyo 103-8405 (JP)**
- **YAMADA, Kumi,**
  **c/o MITSUBISHI PHARMA CORPORATION**
  **Chuo-ku,**
  **Tokyo 103-8405 (JP)**

(74) Representative: **von Kreisler, Alek et al**
**Deichmannhaus am Dom,**
**Postfach 10 22 41**
**50462 Köln (DE)**

# (54) SPECIFIC NAD(P)H OXIDASE INHIBITOR

(57) The present invention provides an agent for inhibiting an excessive effect of NAD(P)H oxidase, which contains a compound that does not substantially inhibit the effect of leukocyte NADPH oxidase but inhibits the effect of NAD(P)H oxidase in tissues other than leukocytes, and a pharmaceutical composition containing said inhibitor.

EP 1 616 576 A1

## Description

### Technical Field

The present invention relates to an inhibitor of overexpression or activation of NAD(P)H oxidase (Nicotinamide adenine dinucleotide (phosphate) oxidase). More particularly, the present invention relates to an agent for inhibiting an excessive effect of NAD(P)H oxidase, which comprises, as an active ingredient, a compound that does not act on NADPH oxidase in leukocytes but shows an inhibitory effect on NAD(P)H oxidase that is overexpressed or activated in tissues other than leukocytes.

### Background Art

It is known that risk factors such as hyperlipidemia, hypertension, diabetes, obesity, aging, smoking etc. trigger the onset of ischemic heart diseases (myocardial infarction or angina etc.), stroke (cerebral infarction, cerebral hemorrhage or subarachnoid hemorrhage etc.), atherosclerosis, peripheral circulation disorder (peripheral arterial occlusion etc.) and the like or aggravate the symptoms, and with regard to the onset and symptomatic aggravation thereof, the importance of oxidative stress caused by reactive oxygen spieces such as superoxide anion ($\bullet O_2^-$) etc. has been drawing close attention in recent years. It has been also reported that the oxidative stress is involved in the growth of cancer (Gene. 2001, 269(1-2), 131), aggravation of Alzheimer's disease (Biochem. Biophys. Res. Commun. 2000, 273(1), 5), side effects caused by anticancer agents (Toxicology. 1999 May 3; 134(1): 51-62), and in the treatment of angina patients with nitrate tolerance (J. Clinical Investigation, 1994, 187-194).

Conventionally, leukocyte has been considered to be the main source of reactive oxygen spieces (e.g., superoxide anion etc.). In recent years, however, production of superoxide anion has also been confirmed in various cell types such as vascular cells, myocytes and the like, and NAD(P)H oxidase, a superoxide anion production enzyme thereof, has been receiving attention (Circ-Res. 2000. 86, 494-501). In the meantime, NADPH oxidase present in leukocytes and NAD(P)H oxidase present in tissues such as vascular cells, myocytes etc. are considered to be nonidentical because they are different in enzyme activity and regulatory mechanism of activity (Cardiovascular Research, 1998, 38, 256-262).

As a compound that inhibits NAD(P)H oxidase activity, diphenyleneiodonium (hereinafter to be indicated as "DPI", see Biochem. Biophys. Res. Commun. 1998, 253, 295), apocynin (WO01/89517), S17834 (Arterioscler-Thromb-Vasc-Biol., 2001, 21:1577) and the like have been reported heretofore. However, these compounds act not only on NADPH oxidase present in leukocytes but also on NAD(P)H oxidase present in tissues other than leukocytes, and do not show tissue specificity.

Patients with chronic granulomatous disease, which is a genetic defect of leukocyte NADPH oxidase, are known to be immunocompromised because superoxide anion production by leukocytes is degraded (J. Leukoc. Biol. 2001, 69, 191). Therefore, when the aforementioned nonspecific NAD(P)H oxidase inhibitor is used as a pharmaceutical agent, side effects due to lower immune function and the like may occur.

### Disclosure of the Invention

The present invention aims at providing a pharmaceutical agent that prevents diseases wherein an excessive effect of NAD(P)H oxidase is a risk factor, or reduces symptoms thereof.

The present inventors have conducted intensive studies and found that the aforementioned problems can be solved by an agent for inhibiting an excessive effect of NAD(P)H oxidase, which does not show an inhibitory effect on leukocyte NADPH oxidase but acts specifically on NAD(P)H oxidase in tissues other than leukocytes, which resulted in the completion of the present invention.

Accordingly, the subject matter of the present invention is an agent for inhibiting an excessive effect of NAD(P)H oxidase, which comprises a compound that does not substantially inhibit the effect of leukocyte NADPH oxidase but inhibits the excessive effect of NAD(P)H oxidase in tissues other than leukocytes, and a pharmaceutical composition for the diseases caused by an effect of excessive NAD(P)H oxidase, which comprises the compound as an active ingredient.

### Brief Description of the Drawings

Fig. 1 shows the effect of the compound on a endothelial-dependent relaxation reaction induced by Ach.

**Best Mode for Embodying the Invention**

The present invention is described in detail in the following.

In the present description, as tissues other than leukocytes, for example, vascular cells, heart, kidney, retina, microglia and the like, as well as tumor cells and the like can be mentioned.

As the vascular cells, for example, endothelial cell, smooth muscle cell, fibroblast, foamy macrophage and the like can be mentioned.

NAD(P)H oxidase means any enzyme that produces superoxide anion using NADH (Nicotinamide adenine dinucleotide) and NADPH (Nicotinamide adenine dinucleotide phosphate) as substrates.

An excessive effect of NAD(P)H oxidase means an effect based on overexpression or activation of NAD(P)H oxidase and, for example, effect induced by risk factors such as diabetes, hypertension, hyperlipidemia, obesity, smoking, heart failure, cardiac hypertrophy, ischemic heart diseases, angioplasty, ischemia-reperfusion in organ transplantation, cancer, dementia, intake of chemicals (e.g., anticancer agent, nitric acid preparation etc.) and the like can be mentioned.

As used herein, overexpression of NAD(P)H oxidase means an expression in an amount beyond necessary for the homeostasis of living organisms, and an expression in an amount beyond necessary for normal tissues from the same origin. As the expression site of NAD(P)H oxidase, for example, tissues of vascular cells, heart, kidney, retina, microglia, tumor cell and the like can be mentioned, but not limited thereto. Accordingly, the inhibitory effect on overexpressing NAD(P)H oxidase means an effect that prevents the overexpressing NAD(P)H oxidase from exerting its enzyme function.

The activation of NAD(P)H oxidase means NAD(P)H oxidase ready to exert a superoxide anion production function, wherein, of the respective subunits constituting NAD(P)H oxidase, $p47^{phox}$, $p40^{phox}$, $p67^{phox}$ and the like have been translocated on the cell membrane. That is, the inhibitory effect on the activated NAD(P)H oxidase means an effect of suppressing an enzyme function of NAD(P)H oxidase ready to develop an enzyme function, and refers to the inhibition of translocation of each subunit constituting NAD(P)H oxidase and/or interaction between respective constituent subunits.

As the diseases caused by an excessive effect of NAD(P)H oxidase, for example, ischemic heart diseases, heart failure, diabetic complications, atherosclerosis, restenosis or reocclusion after angioplasty, disorder after organ implantation, stroke, nitrate tolerance, side effects of anticancer agents, dementia, progression of cancer and the like can be mentioned.

As a compound that does not substantially inhibit the effect of leukocyte NADPH oxidase and inhibits the effect of NAD(P)H oxidase in tissues other than leukocytes, for example, bicyclic pyridazine compounds represented by the following formulas (I)-(VIII) can be mentioned.
The formula (I)

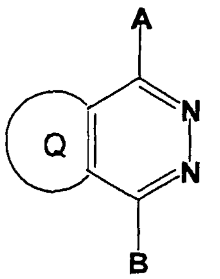

(I)

wherein A is $C_3$-$C_6$ alkyl, $C_5$-$C_7$ cycloalkyl, or phenyl, thienyl, furyl, thiazolyl, phenoxy, $C_7$-$C_9$ phenylalkyl, phenylthio, nitrogen-containing saturated ring group, pyridyl or imidazolyl, each optionally having one or more substituents selected from $C_1$-$C_4$ alkyl, $C_1$-$C_4$ alkoxy and halogen,
B is -NH-D
[D is

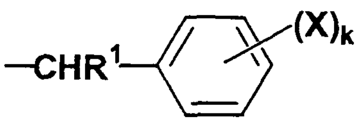

wherein $R^1$ is hydrogen or $C_1$-$C_4$ alkyl, X is halogen, $C_1$-$C_4$ alkyl or $C_1$-$C_4$ alkoxy, and k is an integer of 0 to 3, when k is an integer of 2 or more, multiple Xs may be the same or different,

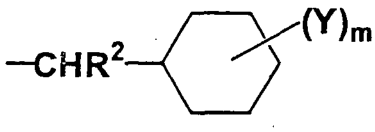

wherein $R^2$ is hydrogen or $C_1$-$C_4$ alkyl, Y is $C_1$-$C_4$ alkyl or $C_{1-}C_4$ alkoxy, and m is an integer of 0 to 6, when m is 2 or more, multiple Ys may be the same or different, and any two Ys may be joined to form optionally branched $C_1$-$C_6$ alkylene,

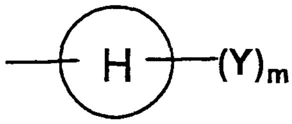

wherein ring H is $C_5$-$C_7$ cycloalkyl, and Y and m are as defined above,

-$CHR^3R^4$

wherein $R^3$ is $C_1$-$C_5$ alkyl, and $R^4$ is $C_5$-$C_8$ cycloalkyl or thienyl,
or $C_3$-$C_8$ alkyl]
or

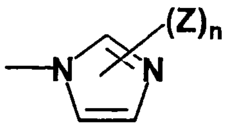

wherein Z is $C_1$-$C_4$ alkyl or phenyl, and n is an integer of 0 to 2, when n is 2, these Zs may be different, and
Q is a benzene ring, a furan ring or a thiophene ring optionally substituted by $C_1$-$C_4$ alkyl.

The compound represented by the formula (I) can be produced according to, for example, a method described in Japanese patent No. 2730421.

The formula (II)

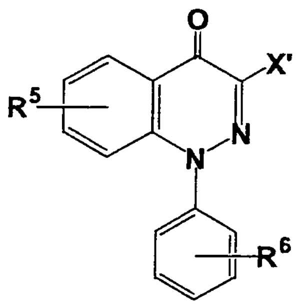

(II)

wherein $R^5$ and $R^6$ are each independently hydrogen, $C_1$-$C_6$ alkyl, $C_1$-$C_6$ alkoxy, halogen, cyano, nitro, amino, trifluoromethyl or carboxyl, and X' is -$COOR^7$ ($R^7$ is hydrogen or optionally substituted $C_1$-$C_6$ alkyl), -$CONH_2$, -CN, -$COR^8$ ($R^8$ is optionally substituted $C_1$-$C_6$ alkyl or optionally substituted aryl), -$NH_2$, -$NO_2$ or -$OR^7$ ($R^7$ is as defined above).

The compound represented by the formula (II) can be produced according to, for example, the methods described in J. Chem. Soc. Chem. Commun., 1974, 752; Synthesis 1983, 52; EP-197226A; US Patent No. 4729782; WO93/09098, and the like.

The formula (III)

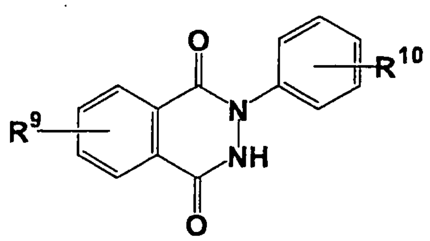

(III)

wherein $R^9$ and $R^{10}$ are each independently hydrogen, $C_1$-$C_6$ alkyl, $C_1$-$C_6$ alkoxy, halogen, cyano, nitro, amino, trifluoromethyl or carboxyl.

The compound represented by the formula (III) can be produced according to, for example, the methods described in Tetrahedron Lett., 37, 1996, 24, 4145, and the like.

The formulas (IV) and (V)

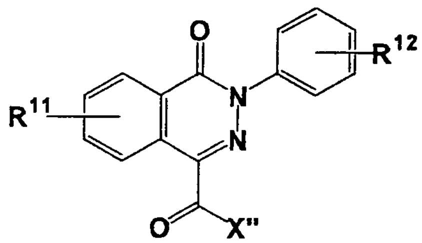

(IV)

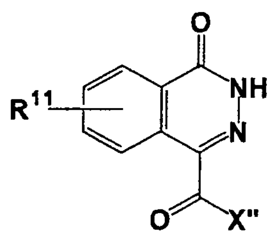

(V)

wherein $R^{11}$ and $R^{12}$ are each independently hydrogen, $C_1$-$C_6$ alkyl, $C_1$-$C_6$ alkoxy, halogen, cyano, nitro, amino, trifluoromethyl or carboxyl, and X" is $-OR^{13}$ ($R^{13}$ is hydrogen, $C_1$-$C_6$ alkyl or aryl) or $-NR^{14}R^{15}$ ($R^{14}$ and $R^{15}$ are each independently hydrogen, $C_1$-$C_6$ alkyl or aryl.

The compound represented by the formula (IV) can be produced according to, for example, the methods described in Heterocycles (1981), 16(1), 25-30, and the like.

The compound represented by the formula (V) can be synthesized according to the methods described in literatures such as Shenyang Yaoke Daxue Xuebao (2001), 18(2), 106-109; Chem. Pharm. Bull. (1980), 28(9), 2763-9; Heterocycles (1981), 16(1), 25-30, and the like.

The formulas (VI), (VII) and (VIII)

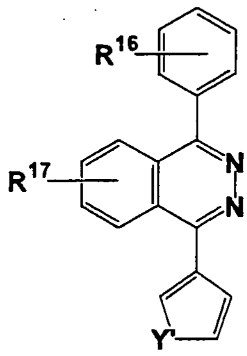

(VI)

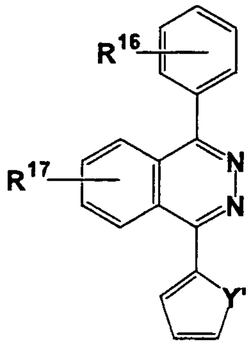

(VII)

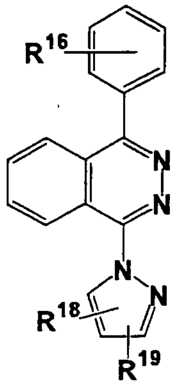

(VIII)

wherein $R^{16}$ and $R^{17}$ are each independently hydrogen, $C_1$-$C_6$ alkyl, alkoxy, halogen, cyano, nitro, amino, trifluoromethyl or carboxyl, $R^{18}$ and $R^{19}$ are each independently hydrogen or $C_1$-$C_6$ alkyl, and Y' is oxygen or sulfur.

The compound represented by the formula (VI) can be produced according to, for example, methods described in JP-2001-335476-A, and the like.

The compound represented by the formula (VII) can be produced according to, for example, the methods described in Tetrahedron Lett. (1986), 27(7), 869-872, and the like.

The compound represented by the formula (VIII) can be produced according to, for example, the methods described in Pharmazie, 46, 2, 1991, 105-8 and the like.

Of these, a bicyclic pyridazine compound represented by the formula (I), (II) or (VIII), particularly a bicyclic pyridazine compound wherein a phenyl group optionally having substituents is bonded at the peri-position thereof and nitrogen atom or oxygen atom is bonded at the p-position thereof is preferable.

As the pharmacologically acceptable salt of the compound represented by the formula (I)-(VIII), for example, acid addition salts such as salts with inorganic acids (e.g., hydrochloric acid, sulfuric acid, hydrobromic acid, phosphoric acid etc.); salts with sulfonic acids (e.g., methanesulfonic acid, paratoluenesulfonic acid etc.); salts with carboxylic acids (e.g., acetic acid, oxalic acid, citric acid, malic acid, fumaric acid etc.) and the like: base addition salts such as salts with metals (e.g., sodium, potassium, magnesium etc.); ammonium salt ; salts with organic amines (e.g., ethanolamine, 2-amino-2-methyl-1-propanol etc.) and the like can be used.

Since the inhibitor of an excessive effect of NAD(P)H oxidase of the present invention (hereinafter sometimes to be indicated as an "NAD(P)H oxidase inhibitor") does not act on leukocyte NADPH oxidase and normal NAD(P)H oxidases other than leukocyte NAD(P)H oxidase, it is extremely effective for treating diseases caused by excessive effect of NAD(P)H oxidase, such as ischemic heart diseases, arteriosclerotic diseases, stroke, diabetic complications and the like or symptoms thereof, particularly for the prophylaxis of the aforementioned diseases associated with lowered immune function or remission of symptoms thereof.

A pharmaceutical composition comprising an NAD(P)H oxidase inhibitor of the present invention can be prepared by mixing a compound that does not substantially inhibit the effect of leukocyte NADPH oxidase and inhibits the effect of NAD(P)H oxidase in tissues other than leukocytes and a conventionally used preparation carrier at a suitable ratio, and processing by a conventional method. In addition, the dosage form only needs to be appropriately selected depending on the administration method. For example, as a preparation for oral administration, tablet, granule, subtle granules, powder, hard capsule, soft capsule, syrup, liquid, emulsion, suspension, elixir and the like can be mentioned, and as a parenteral preparation, injection, adhesive agent, suppository and the like can be mentioned.

Moreover, since the pharmaceutical composition of the present invention prevents diseases induced by various risk factors or reduces symptoms thereof, it can be administered concurrently with hypolipidemic agent, antihypertensive agent, hypoglycemic agent, vasodilator, antiplatelet agent, anticoagulant, brain protective agent, anticancer agent, diuretic agent, cardiotonic agent, analgesic agent, antiedemic agent, thrombolytic agent, immunosuppressant, steroid, vitamins or antioxidant, or administered separately therefrom, or administered sequentially therewith.

The therapeutically effective amount of the NAD(P)H oxidase inhibitor to be contained in the pharmaceutical composition of the present invention can be appropriately determined depending on the age, conditions and the like of the patients. Generally, about 0.001 to 100 mg/kg is preferably administered parenterally or about 0.001 to 100 mg/kg is preferably administered orally to an adult.

## Examples

While the present invention is explained in detail in the following by referring to Examples, the present invention is not limited to the following Examples as long as the scope of the invention is not deviated.

The structural formulas of the compounds used for each test mentioned below are shown below. Compound A and compound B are compounds represented by the formula (I) or a salt thereof, compound C to compound G are compounds represented by the formula (II), and compound H is a compound represented by the formula (VIII).

| | structural formula | | structural formula |
|---|---|---|---|
| compound A | 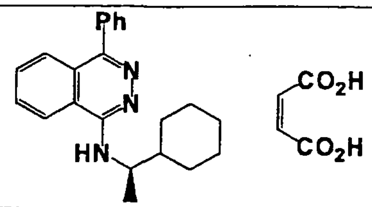 | compound B | 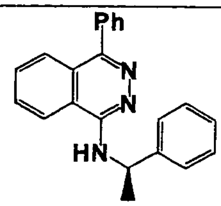 |
| compound C | 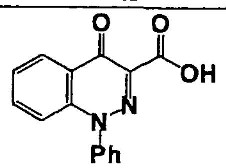 | compound D | 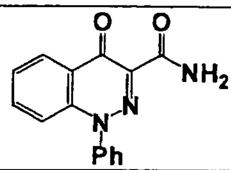 |
| compound E | 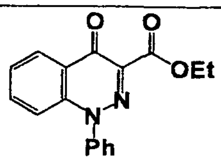 | compound F | 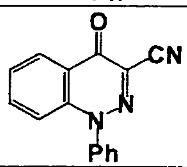 |
| compound G | 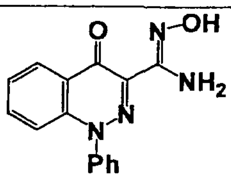 | compound H | 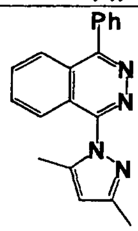 |

**Experimental Example 1** NAD(P)H oxidase inhibitory effect

(Method)

44 mM glucose was added to Human Umbilical Vein Endothelial Cells (Bio Whittaker, hereinafter to be indicated as "HUVEC"), and cells were cultured for 8 days. Then, a lysis buffer containing a surfactant, Triton-X Nonidet P-40, was added, and interleukin-8 (hereinafter to be indicated as "IL-8") in the cell lysate, which had been produced by NAD(P)H oxidase was measured by an enzyme-linked immuno solvent assay. The test compound was added one day before glucose addition.

The inhibitory rate was calculated from the following formula:

$$\text{inhibitory rate } (\%) = 100 - [(A-C)/(B-C)] \times 100$$

wherein A is IL-8 production of a cell cultured in the presence of 44 mM glucose with addition of the test compound, B is IL-8 production of a cell cultured in the presence of 44 mM glucose without addition of the test compound, and C is IL-8 production of a cell cultured in the absence of 44 mM glucose and the test compound. The $IC_{50}$ value was calculated from the dose-effect line drawn by the least squares method based on the obtained inhibitory rate. The results are shown in Table 1.

Table 1 IL-8 production inhibitory effect

| | inhibitory rate (%) | | | $IC_{50}$ value (μM) |
|---|---|---|---|---|
| | test compound concentration (μM) | | | |
| | 0.01 | 0.1 | 1 | |
| compound A | 39.5 | 59.8 | 74 | 0.0063 |
| compound B | 63.8 | 77.8 | 72.0 | <0.01 |
| compound C | -2.5 | 53.7 | 97.7 | 0.0154 |
| compound D | 45.4 | 98.3 | 100 | 0.0111 |
| compound E | 96.3 | ND | 91.0 | <0.01 |
| compound F | 0 | 0 | 54.5 | 0.943 |
| compound G | 39.7 | 43.9 | 57.4 | 0.219 |
| compound H | 27.6 | 49.9 | 83.0 | 0.069 |
| ND: no data | | | | |

**Experimental Example 2** NAD(P)H oxidase inhibitory effect

Streptozotocin (40 mg/kg) dissolved in 0.05 M citric acid buffer (pH 4.5) was administered into the tail vein of male Wistar rats (Japan SLC) to prepare diabetic rats. At 1 week to 8 weeks from the intravenous injection of streptozotocin, blood was drawn from the tail vein using a capillary treated with heparin, which was immediately cooled with ice and centrifuged at 3000 rpm, 15 min, 4°C to give serum. The blood glucose level was measured by enzyme assay using a glucose measurement kit "GLU neo sinotest" (sinotest) and a microplate reader (SPECTRA MAX250, Molecular Devices).

The test compound (10 mg/kg) was orally administered to diabetic rats once a day for 3 days. The NAD(P)H oxidase activity was measured by the following method, which was modified by the method of the David. G. Harrison et al. (J. Clin. Invest. 91, 2546-2551, 1993), at 4 days from the start of the test, with superoxide anion production in aorta as an index.

To be specific, normal rat or diabetic rat free of the above-mentioned treatment was exsanguinated from the abdominal aorta, their thoracic aorta was removed. The removed thoracic aorta was immersed in Krebs-Hepes buffer and marginal tissues were removed to prepare an about 5 mm ring specimen. This ring specimen was preincubate in Krebs-Hepes buffer at 37°C for 10 min and transferred to Krebs-Hepes buffer containing 0.25 mM lucigenin (SIGMA). 500 μM NADH (SIGMA) was added and chemiluminescence value (chemiluminescence: unit RLU: relative light units) was measured using a luminometer (MULTI-BIOLUMAT LB9505C, Warrack·Berthold) at 37°C for 10 min. The total value of chemiluminescence was calculated from the area under the curve (hereinafter to be referred to as "AUC") drawn by plotting the chemiluminescence value (vertical axis) relative to the measurement time (horizontal axis). The measured total value of chemiluminescence was divided by the wet weight of the vascular ring specimen and standardized to the superoxide anion production per aorta unit weight.

The inhibitory rate (%) was calculated from the following formula:

$$\text{inhibitory rate } (\%) = 100 - [(A - C)/(B - C)] \times 100$$

wherein A was the standardized value of chemiluminescence of diabetic rat administered with the test compound, B was the standardized value of chemiluminescence of diabetic rat without administration of the test compound, and C was the standardized value of chemiluminescence of normal rat without administration of the test compound. The results are shown in Table 2 and Table 3.

Table 2 NAD(P)H oxidase activity in diabetic rat aorta

| | chemiluminescence (×$10^5$ RLU/wet weight of aorta) | inhibitory rate (%) |
|---|---|---|
| normal rat | 4.4±1.0 | - |

Table continued

| | chemiluminescence ($\times 10^5$ RLU/wet weight of aorta) | inhibitory rate (%) |
|---|---|---|
| diabetic rat | 14.6±3.4 | - |
| compound A administration group | 3.8±0.6 | 105.8 |
| mean ± standard error | | |

Table 3 NAD(P)H oxidase inhibitory effect in diabetic rat aorta

| test compound | inhibitory rate (%) | | | |
|---|---|---|---|---|
| | 0.01 mg/kg | 0.1 mg/kg | 1 mg/kg | 10 mg/kg |
| compound A | ND | ND | 76.9 | 105.8 |
| compound B | ND | ND | ND | 88.5 |
| compound C | 45 | 90 | ND | ND |
| compound D | 44 | 72 | ND | ND |
| ND: no data | | | | |

As shown in Tables 1 - 3, the NAD(P)H oxidase activity of the endothelial cells cultured in the presence of 44 mM glucose and the aorta of diabetic rat was higher than normal and compounds A to H all showed an NAD(P)H oxidase inhibitory effect.

**Experimental Example 3** effect on normal rat NAD(P)H oxidase

The test compound (10 mg/kg) was administered to normal male Wistar rats (Japan SLC) for 3 days, and the production amount of superoxide anion in the aorta was measured in the same manner as in Experimental Example 2.
The inhibitory rate (%) was calculated by the following formula:

$$\text{inhibitory rate (\%)} = 100 - (A/B) \times 100$$

wherein A is the standardized chemiluminescence value of the rats administered with the test compound, and B is the standardized chemiluminescence value of the rats without administration of the test compound (control group).
The results are shown in Table 4.

Table 4 NAD(P)H oxidase inhibitory effect in normal rat aorta

| test compound | chemiluminescence ($\times 10^5$ RLU/wet weight of aorta) | inhibitory rate (%) |
|---|---|---|
| control group | 3.96±2.9 | - |
| compound A | 3.89±3.0 | 1.8 |
| compound B | 4.01±6.0 | -1.3 |
| mean ± standard error | | |

The compounds A and B did not affect the superoxide anion production in normal rat.

**Experimental Example 4** aorta NAD(P)H oxidase inhibitory effect

The abdominal aorta removed in the same manner as in Experimental Example 2 was immersed in Krebs-Hepes buffer and the marginal tissues were removed to prepare an about 5 mm ring specimen. This ring specimen was preincubated in Krebs-Hepes buffer containing the test compound (compound A, compound B or DPI (SIGMA)) at 37°C for 10 min, and then transferred to Krebs-Hepes buffer containing 0.25 mM lucigenin. NADH (500 μM) and the test compound were added and the chemiluminescence value was measured at 37°C for 10 min.

The total value of chemiluminescence was calculated from AUC of the curve drawn by plotting the chemiluminescence value (vertical axis) relative to the measurement time (horizontal axis). The obtained chemiluminescence value was divided by the wet weight of the vascular ring specimen and standardized to the superoxide anion production per aorta unit weight.

The inhibitory rate (%) was calculated by the following formula:

$$\text{inhibitory rate (\%)} = 100 - (A/B) \times 100$$

wherein A was the standardized chemiluminescence value of ring specimen added with the test compound and B was the standardized chemiluminescence value of ring specimen (control) without addition of the test compound.

The results are shown in Table 5.

Table 5 NAD(P)H oxidase inhibitory effect in diabetic rat aorta

| test compound | chemiluminescence ($\times 10^5$ RLU/wet weight of aorta) | inhibitory rate (%) |
|---|---|---|
| control | 9.3±0.5 | - |
| compound A (0.1 μM) | 9.1±1.7 | 2.2 |
| compound A (1 μM) | 9.2±1.7 | 1.1 |
| DPI (200 μM) | 5.2±1.1 | 45.1 |
| mean ± standard error | | |

While compound A did not show an NAD(P)H oxidase inhibitory effect, DPI, a nonspecific inhibitor, showed an NAD(P)H oxidase inhibitory effect.

**Experimental Example 5** effect on leukocyte NADPH oxidase

Blood was drawn from the abdominal aorta of normal male Wister rats (Japan SLC) and immediately mixed with an ice-cold lysis buffer at 42 mL of the buffer per 3 mL of the blood. After 5 min, the mixture was centrifuged at 4°C, 1100 rpm for 5 min. The precipitate was suspended in an ice-cold Lysis buffer, and centrifuged again at 4°C, 1100 rpm for 5 min. This operation was repeated 3 times to obtain leukocytes. The obtained leukocytes were suspended in Krebs-Hepes buffer and the number of the cells was adjusted to $1\times10^6$/ml. 0.25 mM Lucigenin and the test agent were added, Phorbol 12-myristate 13-acetate (hereinafter to be indicated as "PMA", SIGMA) was further added, and the chemiluminescence value was measured at 37°C for 10 min.

The inhibitory rate (%) was calculated by the following formula:

$$\text{inhibitory rate (\%)} = 100 - [(A-C)/(B-C)] \times 100$$

wherein A is the total chemiluminescence value of the leukocyte suspension with the addition of the test compound, B is the total chemiluminescence value of the leukocyte suspension (control) without addition of the test compound, and C is the total chemiluminescence value of the leukocyte suspension without stimulation with PMA.

The results are shown in the following Table 6.

Table 6 effect on leukocyte NADPH oxidase

| | inhibitory rate (%) |
|---|---|
| compound A 1 μM | 2.4 ± 13.6 |
| compound B 1 μM | 11.7 ± 24.2 |
| DPI 200 μg/mL | 106.7 ± 0.2 |
| mean ± standard error | |

While compounds A and B did not show an inhibitory effect on leukocyte NADPH oxidase, DPI, a nonspecific inhibitor, showed an inhibitory effect on leukocyte NADPH oxidase.

From the results of Experimental Examples 1 to 5, it is clear that the bicyclic pyridazine compounds represented by the formulas (I) to (VIII) do not show an inhibitory effect on leukocyte NADPH oxidase but show an inhibitory effect on the excessive effect of NAD(P)H oxidase in tissues other than leukocytes.

**Experimental Example 6** effect on inhibition of NAD(P)H oxidase expression by oxidized low-density lipoprotein (hereinafter to be indicated as "LDL")

HUVEC was seeded in a 24-well collagen-coated plate. Compound A was added, and oxidized LDL (100 μg/mL) prepared from the blood obtained from normal healthy volunteers was added 24 hr later.
At 24 hr after the oxidized LDL addition, total RNA was extracted using MgExtractor (TOYOBO) and DNase I treatment (Nippon gene) was conducted.
The concentration of the total RNA was calculated by measuring absorbance ($OD_{260}$) of a fraction therefrom.
Using the obtained total RNA (100 ng), RT (reverse transcription) reaction (ABI) was carried out to synthesis cDNA.
Using the synthesized cDNA (10 μL), PCR (polymerase chain reaction) was carried out (ABI), and expression amounts of $p22^{phox}$ which is a major component of NAD(P)H oxidase, and β-actin as an internal standard were measured.
The expression amount of $p22^{phox}$ was standardized to the expression amount of β-actin and expressed based on the expression amount of ($p22^{phox}$)/(β-actin) of normal as 100%.

Table 7 effect on $p22^{phox}$ expression

| normal group | Oxidized LDL addition group (control group) | Oxidized LDL+compound A (0.1 μM) addition group |
|---|---|---|
| 100.00±2.01# | 165±8.05 | 116.64±16.61* |
| #:p<0.05 (vs control group by t-test)<br>*:p<0.05 (vs control group by t-test)<br>mean ± standard error | | |

The $p22^{phox}$ expression of HUVEC increased by about 1.6-fold by the addition of oxidized LDL. In contrast, $p22^{phox}$ expression of the compound A addition group was only 1.2-fold of that of the normal group, and it is clear that compound A inhibited increase in the $p22^{phox}$ expression by the addition of oxidized LDL.

**Experimental Example 7** atherosclerosis inhibitory effect

A 0.67% cholesterol-containing feed (40 g/kg/day) and compound A were given to male NZW rabbits (Japan SLC) for 12 weeks. Blood was drawn from the auricular vein using a heparinized capillary tube, and centrifuged at 3000 rpm for 10 min. The total cholesterol (hereinafter to be indicated as "TC") value of the obtained plasma was measured by an enzyme assay using a measurement kit.
The results are shown in Table 9.
After 12 weeks, the carotid artery was peeled and removed with pentobarbital anesthesia, and exsanguinated to remove the aorta. The obtained carotid artery was immediately placed in Krebs buffer, the surrounding tissues were carefully removed, and the ring specimen was prepared trying not to damage endothelial cells. Then, the ring specimen was suspended in a Magnus tank filled with Krebs buffer at 37°C under an air flow of 95% $O_2$-5% $CO_2$ mixed gas. The experiment was conducted after reaching a steady state by loading 2 g tension.
For the measurement of a relaxation response, the specimen was contracted in advance with norepinephrine, and when the contraction reached steady state, acetylcholine was cumulatively added. The vascular relaxation rate was determined based on the contraction induced by 10 μM norepinephrine as 100%.
Using a transducer (NIHON KOHDEN), tension was measured isometrically. The significant difference was tested for by variance analysis with two-way layout (Dunnett's method) relative to the control group.
The removed aorta was divided into arch aorta, thoracic aorta and abdominal aorta, fixed with 10% formalin buffer, photographed, the area of the lipid adhered to the aorta was measured and the proportion of the area of the lipid-deposition area relative to the area of aorta was determined.
The results are shown in Table 8.

Table 8 effect on lipid-deposition in atherosclerosis rabbit model

| | lipid-deposition area (%) | |
|---|---|---|
| | thoracic aorta | abdominal aorta |
| normal feed group | 0.2 ± 0.1## | 2.1 ± 0.7## |
| control group | 93.5 ± 4.0 | 67.6 ± 8.6 |
| compound A (1 mg/kg) | 62.1 ± 10.0 ** | 25.4 ± 6.2 ** |
| compound A (10 mg/kg) | 56.5 ± 10.5 ** | 18.2 ± 2.2 ** |
| ##:p<0.01 (vs control group by Dunnett's test) | | |
| **:p<0.01 (vs control group by t-test) | | |
| mean ± standard error | | |

Table 9 effect on blood lipid at 12 weeks from cholesterol-loading

| | Plasma lipid concentration TC (mg/dL) |
|---|---|
| normal feed group | 34.1 ± 3.3## |
| control group | 2212.8 ±284.1 |
| compound A (1 mg/kg) | 1895.3 ±244.6 |
| compound A (10 mg/kg) | 1887.9 ±103.9 |
| ##: p<0.01 (vs control group by t-test) | |
| mean ± standard error | |

The plasma TC value of the control group increased to about 2000 mg/dL by the administration of a 0.67% cholesterol-containing feed. The plasma TC value did not show a significant lowering effect as compared to the control group.

A concentration reaction curve of acetylcholine (hereinafter to be indicated as "Ach") at $10^{-8}$ to $10^{-5}$ M for the endothelial-dependent vascular relaxation response of carotid artery is shown in Fig. 1.

The relaxation response of the control group by Ach was significantly attenuated as compared to the normal feed group. On the other hand, the relaxation response of the compound A administration group by Ach was significantly improved as compared to the control group.

Compound A significantly inhibited lipid-deposition to the thoracic and abdominal aortas without an improving effect on hyperlipidemia, which is a risk factor of atherosclerosis. The results suggests that compound A inhibits lipid-deposition to tissues and improves vascular endothelial-dependent relaxation reaction even in the presence of high concentration lipid in plasma.

**Experimental Example 8** effect on rat left coronary artery-ligated myocardial infarction model

Male SD rats (Japan SLC) were fixed in the dorsal position with ether anesthesia, and subjected to thoracotomy by vertical incision along the left sternal line according to the method by Selye H. et al. (Angiology, 1960, 11, 398) to expose the heart. The left coronary artery was ligated using a silk thread (No. 4) at 1-2 mm from the basal part, the heart was put back in place and quickly deaerated, and the chest was closed. The rat was confirmed to have myocardial infarction with an electrocardiograph (NIHON KOHDEN). The rat was exsanguinated to death from the abdominal aorta 24 hr after ligation. The heart was removed, and a circular cross-section at the center thereof was incubated in a solution of 1% 2,3,5-triphenyltetrazolium chloride (hereinafter to be indicated as "TTC") in 0.1 M phosphate buffer for 20 min under light shielding. Thereafter, the section was fixed with formalin buffer, traced, and the areas of infarction region (TTC unstained region) and left ventricle were calculated. Immediately after coronary artery ligation, compound A was suspended in 1% CMC solution and orally administered to the rat.

The inhibitory rate was calculated by the following formula:

$$\text{inhibitory rate (\%)} = \frac{\text{infarction rate of compound administration group}}{\text{infarction rate of compound non-administration group}} \times 100$$

$$\text{infarction rate (\%)} = (\text{infarction region area/left ventricle area}) \times 100$$

In addition, compound A (1 mg/kg) was orally administered at 15 min, 1 hr and 3 hr after ligation, and infarction inhibitory effect was examined. The results are shown in the following Tables.

Table 10 effect on rat acute myocardial infarction model

| | infarction rate (%) | inhibitory rate (%) |
|---|---|---|
| non-administration | 40 | - |
| compound A (0.1 mg/kg) | 40 | 0 |
| compound A (0.3 mg/kg) | 17.2 ** | 57 ** |
| compound A (1 mg/kg) | 6.3 ** | 84.3 ** |
| **:p<0.01 (vs compound non-administration group by Dunnett's test) | | |

Table 11 effect on infarction rate by administration after ligation

| | infarction rate (%) | inhibitory rate (%) |
|---|---|---|
| non-administration | 40 | - |
| 15 min later | 10.5 ** | 73.8 |
| 1 hr later | 16.7 ** | 58.3 |
| 3 hr later | 29.3 * | 26.8 |
| *:p<0.05,**:p<0.01 (vs compound non-administration group by Dunnett's test) | | |

While the infarction rate of the left ventricle was about 40% in the compound non-administration group, the compound A administration group showed a significant infarction rate inhibitory effect in the 0.3 mg/kg and 1 mg/kg administration groups.

**Experimental Example 9** effect on rabbit ischemia-reperfusion myocardial infarction model

Male NZW rabbits (Japan SLC) were fixed in the dorsal position and subjected to thoracotomy with pentobarbital anesthesia. A silk thread (No. 4) was applied to the proximal left coronary artery, a polyethylene tube (length 1 cm) was further applied and the left coronary artery was ligated using a thread. After 2 hr from the ligation, the thread on the polyethylene tube was cut to reperfusion. After 4 hr from the reperfusion, the proximal coronary artery was ligated using a silk thread (No. 4), and 3% Evans blue solution (1 mL/kg) was intravenously administered. After 5 min, an excess pentobarbital was administered and the heart and aorta were removed. The heart was cut into a 3 mm-wide annular shape, and the left ventricle area and ischemia region (Evans blue unstained region) were traced.
After incubation in 1% TTC solution for 30 min, an infarction focus (TTC unstained region) was traced. Compound A (1 mg/kg) was administered 1 hr before the ligation.
The ratios of the ischemic region and infarction focus to the left ventricle area were calculated.
A part of the heart was freezed with liquid nitrogen, crushed with a crusher, and homogenated with phosphate buffer. This suspension was centrifuged at 3000 rpm for 10 min, and the supernatant (8 mL) was centrifuged at 15000

rpm for 15 min. The obtained supernatant was mixed with phosphate buffer containing o-dianisidine dihydrochloride, kept at 25°C for 30 min, the absorbance ($OD_{460}$) was measured, and the myeloperoxidase (MPO) activity was determined. The MPO activity was expressed based on the value of the normal group as 100%. The results are shown in Table 12.

Table 12 effect on rabbit myocardial infarction model

| | infarction area/ischemia area | MPO activity in myocyte |
|---|---|---|
| normal group | - | 100% |
| Control group | 58% | 248%## |
| compound A administration group | 14%* | 128%* |
| *:p<0.05 (vs control group by t-test)<br>##:p<0.01 (vs normal group by t-test) | | |

In the control group, the proportion of the infarction area relative to the ischemia region was 58%. In contrast, in the compound A administration group, this proportion was 14%, showing a significant inhibitory effect. In addition, the MPO activity, which is an index of leukocyte infiltration into cardiac muscle, significantly increased in the control group (infarction heart) as compared to the normal group. However, it was significantly inhibited in the compound A administration group as compared to the control group.

**Experimental Example 10** effect on rabbit chronic myocardial infarction (heart failure) model

Male NZW rabbits (Japan SLC) were fixed in the dorsal position and subjected to thoracotomy with pentobarbital anesthesia. A silk thread (No. 4) was applied to the basal part of the left coronary artery, a polyethylene tube (length 1 cm) was further applied and the left coronary artery was ligated using a thread. After 2 hr from the ligation, the thread on the polyethylene tube was cut to reperfusion. At 2 hr from the reperfusion, compound A (1 mg/kg) was administered once, and administered once a day for 12 weeks. After 12 weeks, an excess pentobarbital was administered, the heart was removed, and the weight thereof and the thickness of the left ventricle center wall were measured. The results are shown below.

The compound was evaluated by the mortality rate, heart weight and thickness of left ventricle.

Table 13 effect on cumulative mortality rate

| | 2 weeks after ligation | 4 weeks after ligation | mortality rate |
|---|---|---|---|
| non-administration group | 3/20 | 6/20 | 30% |
| compound A administration group | 0/12 | 1/12 | 8% |

Table 14 effect on heart weight

| | heart weight (g/kg body weight) | thickness (mm) of left ventricle |
|---|---|---|
| normal group | 1.85# | 1.23# |
| non-administration group | 2.35 | 1.01 |
| compound A administration group | 2.05* | 1.17* |
| *:p<0.05 (vs control group by t-test)<br>#:p<0.05 (vs control group by t-test) | | |

In 12 weeks after ischemia-reperfusion myocardial infarction in rabbit, the test compound non-administration group showed an increase in the heart weight, thinning and weakening of the left ventricle, and expansion and enlargement of the heart as observed in heart failure after myocardial infarction. In the compound A administration group, an increase in the heart weight and thinning and weakening of the left ventricle were inhibited.

**Experimental Example 11** effect on cerebral infarction model (ischemic brain disorder model)

Male ICR mice (Japan SLC) were anesthetized with ether and fixed in the dorsal position. The both common carotid arteries were ligated using a thread. The wound was closed, and the mice were released in a cage. The death of the mouse was confirmed every 30 min up to 240 min, and cumulative mortality rate of each group was determined. Compound B was orally administered 1 hr before common carotid artery ligation.

Table 15 effect on cumulative mortality rate

| mortality rate | control group | compound B (1 mg/kg) | compound B (3 mg/kg) | compound B (10 mg/kg) |
|---|---|---|---|---|
| 0- 30 | 22(71.0%) | 15(78.9%) | 11 (57.9%) | 8(40.0%) |
| 31- 60 | 5(87.1%) | 2(84.2%) | 5(84.2%) | 6(70.0%) |
| 61- 90 | 0 | 2(94.7%) | 0 | 2(80.0%) |
| 91-120 | 2(93.5%) | 0 | 0 | 1(85.0%) |
| 121-150 | 0 | 0 | 1(89.5%) | 0 |
| 151-180 | 2(100%) | 0 | 0 | 0 |
| 181-210 | 0 | 0 | 0 | 0 |
| 211-240 | 0 | 0 | 0 | 0 |
| total | 31 | 20 | 19 | 19 |

In the compound B administration group, a mortality rate improving effect was seen in a brain ischemia model by ligation of both carotid arteries.

**Industrial Applicability**

The pharmaceutical composition containing NAD(P)H oxidase of the present invention is extremely useful, because it can be used for the prophylaxis of diseases caused by various risk factors and the remission of symptoms thereof, and causes only a few side effects because of a small influence exerted on the immune function.
This application is based on a patent application No. 2003-103576 filed in Japan, the contents of which are hereby incorporated by reference.

**Claims**

1. An agent for inhibiting an excessive effect of NAD(P)H oxidase, which comprises a compound that does not substantially inhibit the effect of leukocyte NADPH oxidase but inhibits the effect of NAD(P)H oxidase in a tissue other than leukocyte.

2. The agent of claim 1, wherein the tissue other than leukocyte is a tissue of a vascular cell, the heart, the kidney, the retina, the microglia or a tumor cell.

3. The agent of claim 1 or 2, wherein the excessive effect of NAD(P)H oxidase is caused by diabetes, hypertension, hyperlipidemia, obesity, smoking, heart failure, cardiac hypertrophy, ischemic heart diseases, angioplasty or ischemia-reperfusion in organ transplantation.

4. The agent of claim 1 or 2, wherein the excessive effect of NAD(P)H oxidase is caused by cancer or dementia.

5. The agent of claim 1 or 2, wherein the excessive effect of NAD(P)H oxidase is caused by intake of chemicals.

6. The agent of any one of claims 1 to 5, wherein the compound that does not substantially affect leukocyte NADPH oxidase but inhibits an excessive effect of NAD(P)H oxidase in a tissue other than leukocyte is a bicyclic pyridazine compound represented by the following formulas (I) to (VIII) or a pharmacologically acceptable salt thereof:
formula (I)

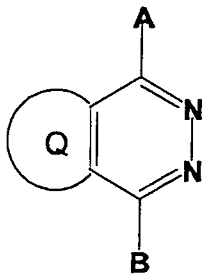

(I)

wherein A is $C_3$-$C_6$ alkyl, $C_5$-$C_7$ cycloalkyl, or phenyl, thienyl, furyl, thiazolyl, phenoxy, $C_7$-$C_9$ phenylalkyl, phenylthio, nitrogen-containing saturated ring group, pyridyl or imidazolyl, each optionally having one or more substituents selected from $C_1$-$C_4$ alkyl, $C_1$-$C_4$ alkoxy and halogen,
B is -NH-D
[D is

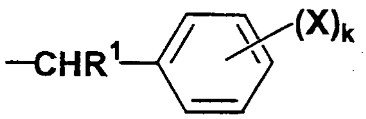

wherein $R^1$ is hydrogen or $C_1$-$C_4$ alkyl, X is halogen, $C_1$-$C_4$ alkyl or $C_1$-$C_4$ alkoxy, and k is an integer of 0 to 3, when k is an integer of 2 or more, multiple Xs may be the same or different,

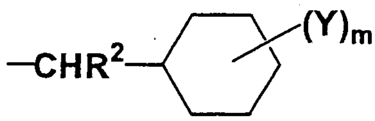

wherein $R^2$ is hydrogen or $C_1$-$C_4$ alkyl, Y is $C_1$-$C_4$ alkyl or $C_{1-}C_4$ alkoxy, and m is an integer of 0 to 6, when m is 2 or more, multiple Ys may be the same or different, and any two Ys may be joined to form optionally branched $C_1$-$C_6$ alkylene,

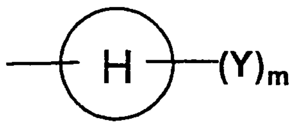

wherein ring H is $C_5$-$C_7$ cycloalkyl, and Y and m are as defined above,

$-CHR^3R^4$

wherein $R^3$ is $C_1$-$C_5$ alkyl, and $R^4$ is $C_5$-$C_8$ cycloalkyl or thienyl,
or $C_3$-$C_8$ alkyl]
or

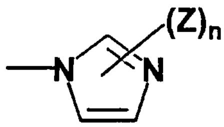

wherein Z is $C_1$-$C_4$ alkyl or phenyl, and n is an integer of 0 to 2, when n is 2, these Zs may be the same or different,

and Q is a benzene ring, a furan ring or a thiophene ring optionally substituted by $C_1$-$C_4$ alkyl,
formula (II)

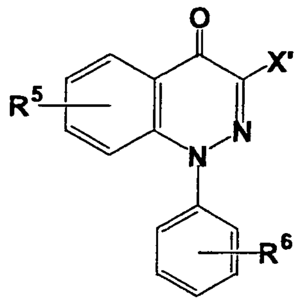

(II)

wherein $R^5$ and $R^6$ are each independently hydrogen, $C_1$-$C_6$ alkyl, $C_1$-$C_6$ alkoxy, halogen, cyano, nitro, amino, trifluoromethyl or carboxyl, and X' is $-COOR^7$ ($R^7$ is hydrogen or optionally substituted $C_1$-$C_6$ alkyl), $-CONH_2$, -CN, $-COR^8$ ($R^8$ is optionally substituted $C_1$-$C_6$ alkyl or optionally substituted aryl), $-NH_2$, $-NO_2$ or $-OR^7$ ($R^7$ is as defined above),
formula (III)

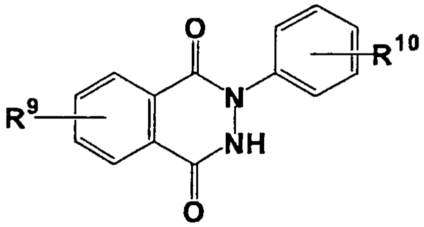

(III)

wherein $R^9$ and $R^{10}$ are each independently hydrogen, $C_1$-$C_6$ alkyl, $C_1$-$C_6$ alkoxy, halogen, cyano, nitro, amino, trifluoromethyl or carboxyl,
formulas (IV) and (V)

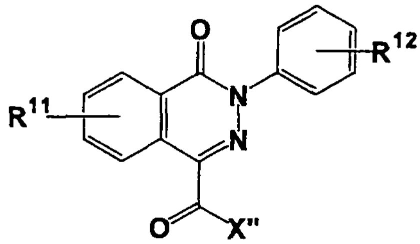

(IV)

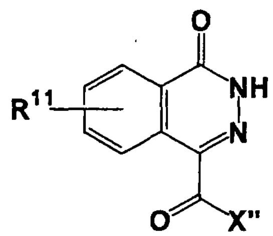

(V)

wherein $R^{11}$ and $R^{12}$ are each independently hydrogen, $C_1$-$C_6$ alkyl, $C_1$-$C_6$ alkoxy, halogen, cyano, nitro, amino, trifluoromethyl or carboxyl, and X'' is $-OR^{13}$ ($R^{13}$ is hydrogen, $C_1$-$C_6$ alkyl or aryl) or $-NR^{14}R^{15}$ ($R^{14}$ and $R^{15}$ are each independently hydrogen, $C_1$-$C_6$ alkyl or aryl,
formulas (VI), (VII) and (VIII)

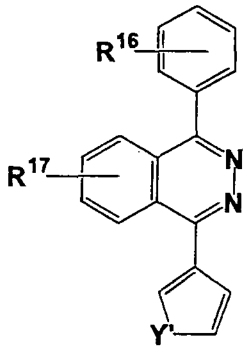
(VI)

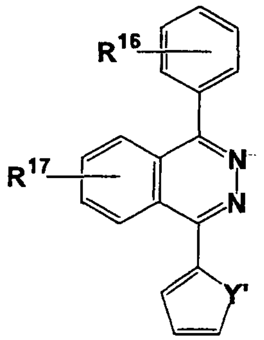
(VII)

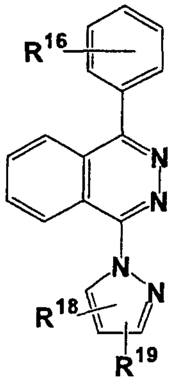
(VIII)

wherein $R^{16}$ and $R^{17}$ are each independently hydrogen, $C_1$-$C_6$ alkyl, alkoxy, halogen, cyano, nitro, amino, trifluoromethyl or carboxyl, $R^{18}$ and $R^{19}$ are each independently hydrogen or $C_1$-$C_6$ alkyl, and Y' is oxygen or sulfur.

**7.** A pharmaceutical composition for the diseases caused by an excessive effect of NAD(P)H oxidase, which comprises the agent of any one of claims 1 to 6 as an active ingredient.

**8.** The pharmaceutical composition of claim 7, which is administered simultaneously with a hypolipidemic agent, an antihypertensive agent, a hypoglycemic agent, a vasodilator, an antiplatelet agent, an anticoagulant, a brain protective agent, an anticancer agent, a diuretic agent, a cardiotonic agent, an analgesic agent, an antiedemic agent, a thrombolytic agent, an immunosuppressant, a steroid, a vitamin or an antioxidant, or administered separately therefrom, or administered sequentially therewith.

FIG. 1

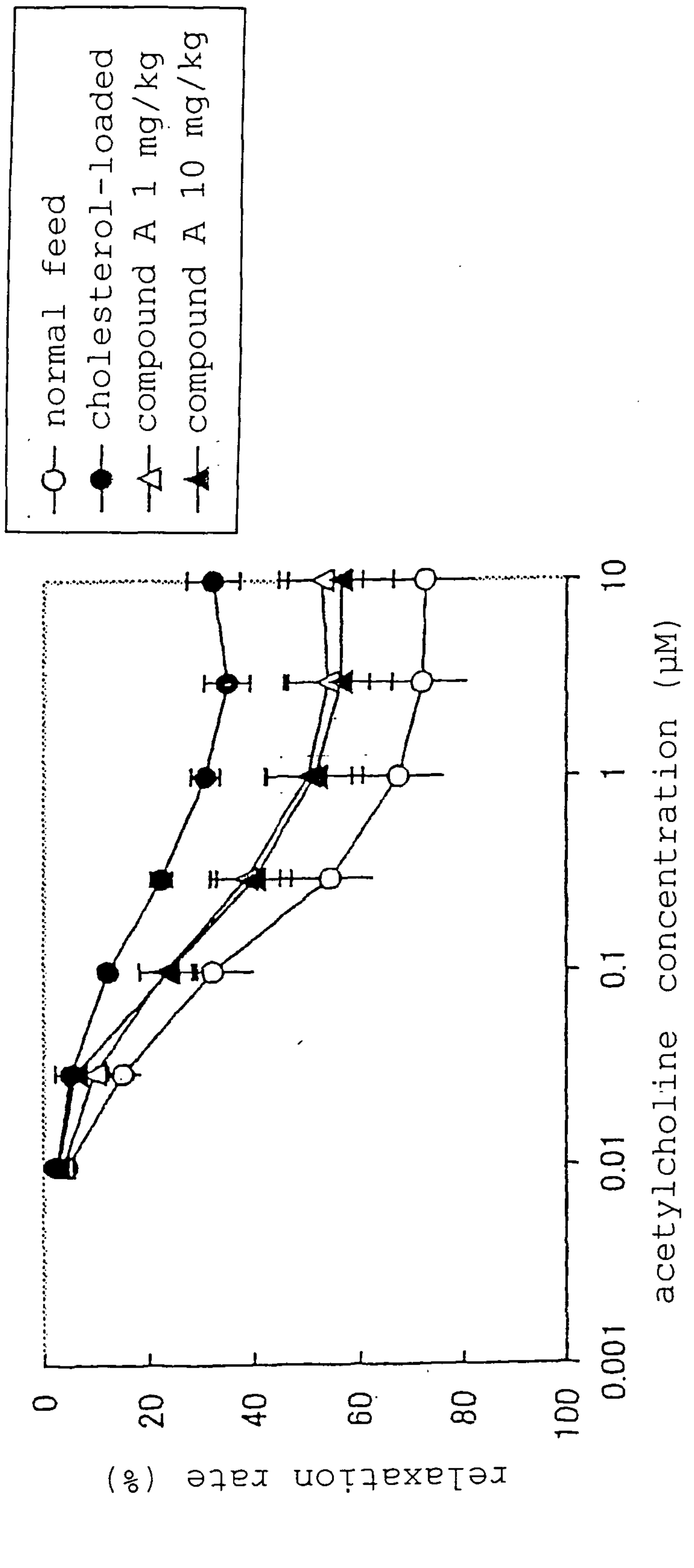
normal feed
cholesterol-loaded
compound A 1 mg/kg
compound A 10 mg/kg
relaxation rate (%)
0
20
40
60
80
100
0.001
0.01
0.1
1
10
acetylcholine concentration (μM)

**INTERNATIONAL SEARCH REPORT**

International application No.
PCT/JP2004/005065

A. CLASSIFICATION OF SUBJECT MATTER
Int.Cl⁷ A61K45/00, 31/502, A61P3/10, 3/04, 3/06, 9/00, 9/10, 9/04, 43/00//C07D403/04, 237/26

According to International Patent Classification (IPC) or to both national classification and IPC

B. FIELDS SEARCHED
Minimum documentation searched (classification system followed by classification symbols)
Int.Cl⁷ A61K45/00, 31/502, A61P3/10, 3/04, 3/06, 9/00, 9/10, 9/04, 43/00, C07D403/04, 237/26

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| X | JP 10-109936 A (Mitsubishi Chemical Corp.), 28 April, 1998 (28.04.98), Full text; particularly, Claims; examples (Family: none) | 1-8 |
| X | JP 8-034734 A (Mitsubishi Chemical Corp.), 06 February, 1996 (06.02.96), Full text; particularly, Claims; examples & EP 682947 A1 & US 5643911 A & CN 1116526 A & CA 2149691 A | 1-8 |
| X | JP 6-135938 A (Mitsubishi Chemical Corp.), 17 May, 1994 (17.05.94), Full text; particularly, Claims; examples & EP 534443 A1 & US 5324727 A & CA 2078699 A | 1-8 |

[X] Further documents are listed in the continuation of Box C.  [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 July, 2004 (13.07.04) | 17 August, 2004 (17.08.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/005065

C (Continuation). DOCUMENTS CONSIDERED TO BE RELEVANT

| Category* | Citation of document, with indication, where appropriate, of the relevant passages | Relevant to claim No. |
|---|---|---|
| X | JP 4-211666 A (Mitsubishi Chemical Corp.), 03 August, 1992 (03.08.92), Full text; particularly, Claims; examples & EP 449203 A1 & US 5089494 A & CA 2039258 A | 1-8 |
| X | JP 2-129180 A (Morishita Pharmaceutical Co., Ltd.), 17 May, 1990 (17.05.90), Full text; particularly, Claims; examples (Family: none) | 1-8 |
| A | JP 62-228067 A (Dainippon Pharmaceutical Co., Ltd.), 06 October, 1987 (06.10.87), Full text; particularly, Claims; examples (Family: none) | 1-8 |

Form PCT/ISA/210 (continuation of second sheet) (January 2004)

INTERNATIONAL SEARCH REPORT

International application No.
PCT/JP2004/005065

<Subject of search>

Claims 1 to 8 relate to an NAD(P)H oxidase hyperfunction inhibitor containing, as the active ingredient, a compound defined by a desired property "substantially not inhibiting NADPH oxidase originating in leukocytes but inhibiting NAD(P)H oxidase originating in tissues other than leukocytes" or a medicinal composition for diseases caused by the hyperfunction of NAD(P)H oxidase. It is recognized that only specific small part of the claimed compounds are supported by the description in the meaning within PCT Article 6 and disclosed therein in the meaning within PCT Article 5.

Even though the common technical knowledge at the point of the application is considered, the scope of compounds having the above property cannot be specified.

Such being the case, the search was made mainly on the compounds A to H which is specifically illustrated as having the above property in the description (in the description, the compounds A and B alone are specifically shown as not inhibiting the leukocyte NADPH oxidase function).

Form PCT/ISA/210 (extra sheet) (January 2004)